(19) **Europäisches Patentamt**

**European Patent Office**

**Office européen des brevets**

(11) **EP 0 485 539 B2**

(12) **NEUE EUROPÄISCHE PATENTSCHRIFT**

(45) Veröffentlichungstag und Bekanntmachung des Hinweises auf die Entscheidung über den Einspruch:
**24.06.1998 Patentblatt 1998/26**

(45) Hinweis auf die Patenterteilung:
**11.10.1995 Patentblatt 1995/41**

(21) Anmeldenummer: **91907467.4**

(22) Anmeldetag: **05.04.1991**

(51) Int. Cl.$^6$: **A61K 7/13**

(86) Internationale Anmeldenummer:
**PCT/EP91/00648**

(87) Internationale Veröffentlichungsnummer:
**WO 91/18582 (12.12.1991 Gazette 1991/28)**

(54) **OXIDATIONSHAARFÄRBEMITTEL AUS EINER EMULSIONSFÖRMIGEN FARBSTOFFTRÄGERMASSE UND EINER EMULSIONSFÖRMIGEN, OXIDATIONSMITTELHALTIGEN ZUSAMMENSETZUNG UND VERFAHREN ZUM OXIDATIVEN FÄRBEN VON HAAREN**

OXIDATIVE HAIR DYES CONSISTING OF AN EMULSIFIED DYE CARRIER SUBSTANCE AND AN EMULSIFIED COMPOSITION CONTAINING AN OXIDIZING AGENT AND PROCESS FOR OXIDATIVE DYEING OF HAIR

COLORANT PAR OXYDATION POUR CHEVEUX OBTENU A PARTIR D'UN SUPPORT COLORANT EMULSIONNE ET D'UNE COMPOSITION EMULSIONNEE CONTENANT UN OXYDANT ET PROCEDE POUR LA COLORATION DES CHEVEUX PAR OXYDATION

(84) Benannte Vertragsstaaten:
**DE ES FR GB IT**

(30) Priorität: **01.06.1990 DE 4017718**

(43) Veröffentlichungstag der Anmeldung:
**20.05.1992 Patentblatt 1992/21**

(73) Patentinhaber:
**Wella Aktiengesellschaft
64295 Darmstadt (DE)**

(72) Erfinder:
• **MAGER, Herbert
CH-1723 Marly (CH)**
• **AEBY, Johann
CH-1723 Marly (CH)**

• **PASQUIER, Gilbert
CH-1724 Praroman (CH)**

(56) Entgegenhaltungen:
EP-A- 308 825            EP-A- 0 216 334
EP-A- 0 258 586         DE-B- 166 100

• **S.T.N. DATENBANK LIFFERANT, (Karlsruhe, DE), DATENBANK CHEMICAL ABSTRACTS, Band 112, Nr. 18, Zusammenfassung Nr. 164733y, & JP,A,01279819 (HOYU CO., LTD) 10. November 1989**
• **K. Schrader, "Grundlagen und Rezepturen der Kosmetika", 2. Auflage (1989), Hüthing Buch Verlag Heidelberg, S. 788, 797-798, 808, 810 und 821-823**

EP 0 485 539 B2

**Beschreibung**

Gegenstand der Erfindung ist ein Mittel zum oxidativen Färben von Haaren, das durch Vermischen einer emulsionsförmigen Farbstoffträgermasse, welche ein 60 bis 100 Gewichtsprozent $C_{10}$- bis $C_{24}$-Fettalkohole aufweisendes Verdickergemisch enthält, mit einer emulsionsförmigen, ein mindestens 75 Gewichtsprozent $C_{10}$- bis $C_{24}$-Fettalkohole aufweisendes Verdickergemisch und ein Oxidationsmittel enthaltenden Zubereitung in einem Mengenverhältnis von 1 : 1,7 bis 1 : 3 erhalten wird, sowie ein Verfahren zum oxidativen Färben von Haaren.

In der Haarfärbepraxis haben Oxidationshaarfärbemittel eine wesentliche Bedeutung erlangt. Die Färbung entsteht im Haarschaft durch die Reaktion bestimmter Entwicklersubstanzen mit bestimmten Kupplersubstanzen in Gegenwart eines geeigneten Oxidationsmittels.

Als Entwicklersubstanzen werden bevorzugt 2,5-Diaminotoluol, 4-Amino-phenol und 1,4-Diamino-benzol verwendet, jedoch haben auch 2,5-Diamino-anisol, 2,5-Diamino-benzylalkohol, 2-(2'-Hydroxyethyl)-1,4-diamino-benzol und 4-Amino-N-(2'-mesylaminoethyl)-anilin eine gewisse Bedeutung erlangt. In bestimmten Fällen kann auch Tetraaminopyrimidin als Entwicklersubstanz eingesetzt werden. Die bevorzugt verwendeten Kupplersubstanzen sind 1-Naphthol, Resorcin, 4-Chlor-resorcin, m-Aminophenol, 5-Amino-o-kresol und Derivate des m-Phenylendiamins wie 2,4-Diamino-phenetol und 2,4-Diamino-anisol.

Durch geeignete Kombinationen von Entwicklersubstanzen mit Kupplersubstanzen läßt sich eine breite Palette verschiedener Farbnuancen erzeugen.

Oxidationshaarfärbemittel bestehen aus zwei Komponenten, die kurz vor dem Gebrauch vermischt und dann auf das zu färbende Haar aufgebracht werden. Die erste Komponente, die Farbträgermasse, enthält die färberisch wirksamen Substanzen. Sie kann als Lösung, Gel oder als Emulsion vorliegen. Die zweite Komponente ist üblicherweise ein wäßriges oder auch pulverförmiges Produkt, in dem ein geeignetes Oxidationsmittel, zum Beispiel Wasserstoffperoxid enthalten ist.

In der Färbepraxis werden Farbträgermassen in Form von Lösungen üblicherweise in Verbindung mit wäßrigen Wasserstoffperoxidlösungen angewandt. Die Vermischung der in Form einer Lösung vorliegenden Farbträgermasse mit der Wasserstoffperoxidlösung kann zum Beispiel in einer Auftrageflasche erfolgen, mit der das gebrauchsfertige Mittel nach dem Mischen auf die Haare aufgebracht wird.

Die auf in Form von Lösungen vorliegenden Farbträgermassen basierenden Oxidationshaarfärbemittel zeigen jedoch eine Reihe von Nachteilen wie eine unzureichende Hautverträglichkeit, eine vermehrte Schädigung des Haares durch einen hohen Ammoniakgehalt und eine unzureichende Deckkraft insbesondere beim Färben von ergrautem Haar.

Zudem ergeben sich für die auf Farbträgermassen, die in Form von Lösungen vorliegen, basierenden Oxidationshaarfärbemittel anwendungstechnische Nachteile, wie das Ablaufen des flüssigen Mittels vom Haar. Diese Nachteile konnten durch den Ersatz der in Form von Lösungen vorliegenden Trägermassen gegen emulsions- oder gelförmige Farbträgermassen nicht aufgehoben werden. Es kam im Gegenteil hinzu, daß sich die emulsions-oder gelförmigen Farbträgermassen mit den flüssigen Wasserstoffperoxidlösungen durch den bestehenden Konsistenzunterschied wesentlich schwerer vermischen lassen.

Die Dokumente EP-A-0 258 586 und EP-A-0 216 334 betreffen Oxidationshaarfärbemittel.

Aufgabe der vorliegenden Erfindung war es daher, ein Mittel zum oxidativen Färben von Haaren sowie ein Haarfärbeverfahren unter Verwendung dieses Mittels zur Verfügung zu stellen, das die geschilderten Nachteile bekannter Mittel zum oxidativen Färben von Haaren nicht aufweist.

Es wurde nunmehr gefunden, daß sich Mittel zum oxidativen Färben von Haaren durch Vermischen einer emulsionsförmigen Farbträgermasse, welche ein 60 bis 100 Gewichtsprozent $C_{10}$- bis $C_{24}$-Fettalkohole aufweisendes Verdickergemisch enthält, mit einer emulsionsförmigen, ein mindestens 75 Gewichtsprozent $C_{10}$- bis $C_{24}$-Fettalkohole enthaltendes Verdickergemisch und ein Oxidationsmittel enthaltenden Zubereitung in einem Mengenverhältnis von 1 : 1,7 bis 1 : 3 herstellen lassen, die gut hautverträglich sind, das Haar durch einen niedrigeren Ammoniakgehalt weniger belasten, und eine gute Deckkraft insbesondere Färbungen von ergrautem Haar aufweisen.

Die erfindungsgemäße Verteilung der Fettalkohole sowohl auf die Farbträgermasse wie auf die das Oxidationsmittel enthaltende Zubereitung bedingt eine ähnliche Konsistenz der beiden Komponenten und eine daraus resultierende leichte Vermischbarkeit der beiden Komponenten zum gebrauchsfertigen Mittel zum oxidativen Färben von Haaren.

Die neue Farbträgermasse besitzt zudem den Vorteil aufgrund ihrer Konsistenz leicht aus der Packung, üblicherweise einer Tube, entnommen werden zu können.

Das gebrauchsfertige neue Mittel zum oxidativen Färben von Haaren weist eine größere Viskosität auf als übliche Oxidationshaarfärbemittel, bei denen die gleiche Menge an konsistenzgebenden Substanzen nur durch die Farbträgermasse in das gebrauchsfertige Mittel eingebracht wird. Das neue Mittel läßt sich zudem problemlos, beispielsweise mit einem Pinsel, auf die Haare auftragen.

Gegenstand der vorliegenden Erfindung ist daher ein 2-Komponenten-Kit zur Herstellung eines Mittels zum oxidativen Färben von Haaren, bestehend aus einer emulsionsförmigen Komponente (A), welche 0,01 bis 12 Gewichtsprozent einer Entwicklersubstanz-Kupplersubstanz-Kombination und 6 bis 30 Gewichtsprozent eines Verdickergemisches enthält, mit einer emulsionsförmigen Komponente (B), welche 1 bis 18 Gewichtsprozent eines Oxidationsmittels und 3 bis 12 Gewichtsprozent eines Verdickergemischs enthält, dadurch gekennzeichnet, daß

a) das in der Komponente (A) enthaltene Verdickergemisch, bezogen auf die Gesamtmenge dieses Verdickergemisches, 60 bis 100 Gewichtsprozent $C_{10}$- bis $C_{24}$-Fettalkohole enthält,
b) das in der Komponente (B) enthaltene Verdickergemisch, bezogen auf die Gesamtmenge dieses Verdickergemisches, 75 bis 100 Gewichtsprozent $C_{10}$- bis $C_{24}$-Fettalkohole enthält und
c) in diesem Kit die Komponente (A) zur Komponente (B) in einem Verhältnis von 1 : 1,7 bis 1 : 3 vorliegt.

Die Viskosität des gebrauchsfertigen Mittels zum oxidativen Färben von Haaren liegt in einem Bereich von 500 - 200 000 mPa · s.

Die in den emulsionsförmigen Komponenten (A) und (B) enthaltenen Fettalkohole sind vorzugsweise $C_{12}$- bis $C_{20}$-Fettalkohole. Beispiele für Fettalkohole, die in den Komponenten (A) und (B) enthalten sein können, sind Cetylalkohol, Stearylalkohol oder deren Gemisch.

Das in der Komponente (A) enthaltene Verdickergemisch kann, bezogen auf die Gesamtmenge dieses Verdickergemisches, bis zu 40 Gewichtsprozent in kosmetischen Emulsionen übliche konsistenzgebende Substanzen wie zum Beispiel $C_{10}$- bis $C_{23}$-Fettsäureester, $C_{10}$- bis $C_{23}$-Fettsäuren, mit 2 bis 8 Mol Ethylenoxid oxethylierte Fettalkohole, Stärke, Vaseline und Wollwachsalkohole enthalten.
Die Anzahl der gegebenenfalls im Verdickergemisch der Komponente (A) enthaltenen Verdicker liegt zwischen 1 und 5, vorzugsweise zwischen 1 und 3.

Besonders bevorzugt enthält das Verdickergemisch der Komponente (A) $C_{10}$- bis $C_{23}$-Fettsäureester mit Glycerin oder Glykolen, wie zum Beispiel Glycerinmonodistearat, zum Beispiel in Form des Handelsproduktes Tegin® der Firma Goldschmidt.

Das in der Komponente (A) enthaltene Verdickergemisch kann zudem, bezogen auf die Gesamtmenge dieses Verdickergemisches, 0,2 bis 25 Gewichtsprozent anionische oder nichtionische Emulgatoren oder deren Gemische enthalten.

Beispiele für anionische Emulgatoren, die in der Kamponente (A) enthalten sein können, sind $C_{10}$- bis $C_{25}$-Fettalkoholsulfate, $C_{10}$- bis $C_{25}$-Fettalkoholethersulfate, Fettalkoholoxethansulfonsäuresalze, Lauryloxethylat mit 10 bis 30 Ethylenoxideinheiten oxethyliert oder oxethyliertes Ricinusöl. Als nicht-ionische Emulgatoren können in der Komponente (A) bevorzugt mit 8 bis 30 Ethylenoxideinheiten oxethylierte $C_{10}$- bis $C_{25}$-Fettalkohole eingesetzt werden, beispielsweise das Handelsprodukt Cremophor A 25 ® (Fa. BASF, Ludwigshafen).

Die Komponente (A) weist einen pH-Wert von 4 bis 13, bevorzugt jedoch 7,5 bis 12,5 auf. Der pH-Wert wird vorzugsweise mit Ammoniak eingestellt. Es können jedoch auch organische Amine, beispielsweise Monoethanolamin, oder anorganische Alkalien wie Natronlauge zur Einstellung des pH-Wertes verwendet werden.

Die Komponente (A) enthält mindestens eine Kupplersubstanz und mindestens eine Entwicklersubstanz sowie gegebenenfalls zusätzlich mit sich selbst kuppelnde Farbvorstufen und direkt auf das Haar auf aufziehende Farbstoffe. Die Entwickler- und

Kupplersubstanzen werden in den Haarfärbemitteln entweder als solche oder in Form ihrer physiologisch unbedenklichen Salze mit anorganischen oder organischen Säuren, wie zum Beispiel als Chlorid, Sulfat, Phosphat, Acetat, Propionat, Lactat oder Citrat, eingesetzt.

Die Kupplersubstanzen werden im allgemeinen in etwa äquimolarer Menge, bezogen auf die verwendeten Entwicklersubstanzen, eingesetzt. Wenn sich auch der äquimolare Einsatz als zweckmäßig erweist, so ist es doch nicht nachteilig, wenn die Kupplersubstanzen in einem gewissen Überschuß oder Unterschuß zum Einsatz kommen. Es ist ferner nicht notwendig, daß die Entwicklerkomponente und die Kupplerkomponente einheitliche Produkte darstellen, vielmehr kann sowohl die Entwicklerkomponente ein Gemisch von bekannten Entwicklersubstanzen als auch die Kupplerkomponente ein Gemisch von bekannten Kupplersubstanzen darstellen.

Die emulsionsförmige Farbträgermasse, Komponente (A), enthält als bekannte Kupplersubstanzen, allein oder im Gemisch miteinander, insbesondere 1-Naphthol, 4-Methoxy-1-naphthol, Resorcin, 4-Chlorresorcin, 4,6-Dichlorresorcin, 2-Methylresorcin, 3-Aminophenol, 3-Amino-6-methylphenol, 4-Hydroxy-1,2-methylendioxybenzol, 4-Amino-1,2-methylendioxybenzol, 4-(β-Hydroxyethylamino)-1,2-methyldioxybenzol, 4-Hydroxyindol, 2,3-Diamino-6-methoxy-pyridin und 5-Amino-2-methylphenol. Weitere geeignete Kupplersubstanzen sind zum Beispiel 2,4-Dihydroxyphenolether wie 2,4-Dihydroxyanisol und 2,4-Dihydroxyphenoxyethanol.

Von den bekannten Entwicklersubstanzen kommen als Bestandteil der erfindungsgemäßen Farbträgermasse vor allem 1,4-Diaminobenzol, 2,5-Diaminotoluol, 2,5-Diaminobenzylalkohol, 3-Methyl-4-aminophenol, 2-(β-Hydroxyethyl)-1,4-diaminobenzol und 4-Aminophenol in Betracht. Zur Haarfärbung bekannte und übliche Oxidationsfarbstoffe, die in der Komponente (A) enthalten sein können, sind unter anderem in dem Buch von E. Sagarin,

"Cosmetics, Science and Technology", Interscience Publishers Inc., New York (1957), Seiten 503 ff. sowie in dem Buch von H. Janistyn, "Handbuch der Kosmetika und Riechstoffe" (1973), Seiten 338 ff. beschrieben.

Die Gesamtmenge der in der Komponente (A) enthaltenen Entwicklersubstanz-Kupplersubstanz-Kombination soll 0,01 bis 12 Gewichtsprozent, insbesondere 0,2 bis 4 Gewichtsprozent, betragen.

Zur Erzielung gewisser Farbnuancen können ferner auch übliche direktziehende Farbstoffe, beispielsweise Triphenylmethanfarbstoffe wie Basic Violet 14 (C.I. 42 510) und Basic Violet 2 (C.I. 42 520), aromatische Nitrofarbstoffe wie 2-Amino-4,6-dinitrophenol, 2-Nitro-4-(β-hydroxyethylamino)-anilin, 2-N-β, -Dihydroxypropylamino-5-(N-methyl,N-hydroxyethyl)-amino-nitrobenzol und 2-Amino-4-nitrophenol, Azofarbstoffe wie Acid Brown 4 (C.I. 14 805) und Acid Blue 135 (C.I. 13 385), Anthrachinonfarbstoffe wie Disperse Violet 4 (C.I. 61 105), Disperse Blue 1 (C.I. 64 500), Disperse Red 15 (C.I. 60 710), Disperse Violet 1 (C.I. 61 100), außerdem 1,4,5,8-Tetraaminoanthrachinon und 1,4-Diaminoanthrachinon, in der Komponente (A) enthalten sein. Die Komponente (A) kann weiterhin auch mit sich selbst kuppelnde Farbvorstufen, wie zum Beispiel 2-Amino-5-methylphenol, 2-Amino-6-methylphenol, 2-Amino-5-ethoxyphenol oder auch 2-Propyl-amino-5-aminopyridin, enthalten.

Die Gesamtmenge der direkt ziehenden Farbstoffe und der mit sich selbst kuppelnden Farbstoffvorstufen beträgt in der Komponente (A) 0,01 bis 6 Gewichtsprozent, vorzugsweise 0,2 bis 4 Gewichtsprozent.

Die Gesamtmenge aller Farbstoffe, also der Entwicklersubstanz-Kupplersubstanz-Kombnation, der mit sich selbst kuppelnde Farbstoffvorstufen und der direktziehenden Farbstoffe, in der Komponente (A) soll 0,1 bis 14 Gewichtsprozent, vorzugsweise 0,2 bis 8 Gewichtsprozent betragen.

Darüberhinaus können in der Komponente (A) Antioxidantien wie Ascorbinsäure, Resorcin oder Natriumsulfit und Komplexbildner für Schwermetalle, beispielsweise Ethylendiamintetraacetat und Nitriloessigsäure in einer Menge von bis zu 0,5 Gewichtsprozent enthalten sein. Parfümöle können in der erfindungsgemäßen Farbträgermasse bis zu einer Menge von 1 Gewichtsprozent enthalten sein. Die Komponente (A) kann zudem Netzmittel, Emulgatoren, Pflegestoffe, kationische Harze und andere übliche Zusatzstoffe enthalten.

Der in der emulsionsförmigen, oxidationsmittelhaltigen Komponente (B) enthaltene Fettalkohol ist vorzugsweise ein C$_{10}$- bis C$_{20}$-Fettalkohol. Beispiele für derartige Fettalkohole, die in der Komponente (B) enthalten sein können, sind Cetylalkohol, Stearylalkohol oder deren Gemisch.

Das in der Komponente (B) enthaltene Verdickergemisch kann zusätzlich zu den C$_{10}$- bis C$_{24}$-Fettalkoholen andere in kosmetischen Mitteln übliche Verdicker, beispielsweise mit 2 bis 6 Einheiten Ethylenoxid oxethylierte C$_{10}$- bis C$_{25}$-Fettalkohole enthalten.

Das in der Komponente (B) enthaltene Verdickergemisch kann, bezogen auf die Gesamtmenge dieses Verdickergemisches, einen Gehalt von 0,2 bis 25 Gewichtsprozent an anionischen oder nichtionischen Emulgatoren oder deren Gemisch aufweisen. Beispiele für Emulgatoren die in der Komponente (B) enthalten sein können sind C$_{10}$- bis C$_{25}$-Fettalkoholsulfate oder -sulfonate, C$_{10}$- bis C$_{25}$-Fettalkoholethersulfate, mit 8 bis 30 Mol Ethylenoxid ethoxylierte C$_{10}$- bis C$_{25}$-Fettalkohole, Cholesterin oder Wollwachsalkohole.

Die Komponente (B) enthält 1 bis 18 Gewichtsprozent, besonders bevorzugt jedoch 4 bis 14 Gewichtsprozent eines Oxidationsmittels. Als Oxidationsmittel zur Entwicklung der Haarfärbung kommen hauptsächlich Wasserstoffperoxid, beziehungsweise dessen Additionsverbindungen an Harnstoff, Melamin und Natriumborat in Betracht. Besonders bevorzugt enthält die Komponente (B) 1 bis 18 Gewichtsprozent Wasserstoffperoxid.

Das durch Mischen der Komponente (A) mit der Komponente (B) entstehende gebrauchsfertige Mittel zum oxidativen Färben der Haare kann sauer, neutral oder alkalisch eingestellt sein. Der pH-Wert des erfindungsgemäßen Mittels zum oxidativen Färben der Haare liegt in einem Bereich von 7,5 bis 12,0, bevorzugt zwischen 9,5 und 10,2.

Die vorstehenden Gewichtsprozentangaben sind, sofern nichts anderes angegeben ist, jeweils auf die Gesamtmenge der Komponente (A), beziehungsweise auf die Gesamtmenge der Komponente (B), bezogen.

Bei der Anwendung des zuvor beschriebenen Oxidationshaarfärbemittels nach dem erfindungsgemäßen Verfahren vermischt man die emulsionsförmige Farbträgermasse (Komponente (A)) unmittelbar vor dem Gebrauch in einem Gewichtsverhältnis von 1 : 1,7 bis 1 : 3, vorzugsweise 1 : 2, mit der oxidationsmittelhaltigen Emulsion (Komponente (B)) und trägt eine für die Haarfärbung ausreichende Menge, je nach Haarfülle, im allgemeinen 90 bis 160 g, dieses gebrauchsfertigen Oxidationshaarfärbemittels auf das Haar auf. Man läßt das Gemisch bei 15 bis 50 Grad Celsius etwa 10 bis 45 Minuten, vorzugsweise 30 Minuten, lang auf das Haar einwirken, spült sodann das Haar mit Wasser aus und trocknet. Gegebenenfalls wird das Haar vor dem Trocknen gewaschen und nachgespült.

Die nachfolgenden Beispiele sollen den Gegenstand der Erfindung näher erläutern.

**Beispiele**

**Beispiel 1: Oxidationshaarfärbemittel**

emulsionsförmige Farbträgermasse (Komponente A):

    8,00 g Cetylstearylalkohol
    1,84 g Natriumlaurylalkoholdiglykolethersulfat,
    28 %ige wäßrige Lösung
    0,50 g Natriumsulfit, wasserfrei
    1,00 g 2,5-Diaminotoluolsulfat
    0,50 g Resorcin
    0,08 g m-Aminophenol
    6,19 g Ammoniak, 25 %ige wäßrige Lösung

81,89 g Wasser
100,00 g

Wasserstoffperoxid-Emulsion (Komponente B):

6,00 g Cetylstearylalkohol
0,15 g Cholesterin
2,40 g Natriumlauryldiglykolethersulfat,
28 %ige wäßrige Lösung
9,00 g Wasserstoffperoxid, 50 %ige wäßrige Lösung
82,45 g Wasser
100,00 g

Der pH-Wert der Komponente (B) des Beispiels 1 ist mit verdünnter Phosphorsäure auf 2,5 eingestellt.

Man vermischt vor dem Gebrauch 40 g der emulsionsförmigen Farbträgermasse mit 80 g der Wasserstoffperoxid-Emulsion, entsprechend einem Mischungsverhältnis von 1 : 2, trägt 120 g des gebrauchsfertigen Oxidationshaarfärbemittels auf ergraute, menschliche Haare auf und läßt es 20 Minuten lang bei Raumtemperatur einwirken. Danach wird das Haarfärbemittel mit Wasser ausgespült und das Haar getrocknet. Das so behandelte Haar ist vom Ansatz bis zu den Haarspitzen gleichmäßig dunkelblond gefärbt. Das erfindungsgemäße Mittel zum oxidativen Färben von Haaren weist eine Viskosität von 1700 m Pa·s auf und läßt sich problemlos mit dem Pinsel auftragen, tropft nicht ab und ergibt eine Färbung von großer Deckkraft.

Alle in dieser Anmeldung angegebenen Viskositäten wurden mit der Haake-Viskowaage bei 20 Grad Celsius (Stab II, Auflagegewicht 5 g) bestimmt.

### Beispiel 2: Oxidationshaarfärbemittel

emulsionsförmige Farbstoffträgermasse (Komponente A):

10,000 g Cetylstearylalkohol
1,000 g Wollwachsalkohole
2,000 g Natriumlaurylalkoholdiglykolethersulfat,
28 %ige wäßrige Lösung
0,500 g Natriumsulfit, wasserfrei
1,350 g 2,5-Diaminotoluolsulfat
0,720 g Resorcin
0,056 g m-Aminophenol
0,028 g m-Phenylendiamin
7,282 g Ammoniak, 25 %ige wäßrige Lösung
77,064 g Wasser
100,000 g

Wasserstoffperoxid-Emulsion (Komponente B):

6,00 g Cetylstearylalkohol
0,15 g Cholesterin
2,40 g Natriumlauryldiglykolethersulfat,

28 %ige wäßrige Lösung
9,00 g Wasserstoffperoxid, 50 %ige wäßrige Lösung
82,45 g Wasser
100,00 g

Der pH-Wert der Komponente (B) des Beispiels 2 wurde mit verdünnter Phosphorsäure auf 2,5 eingestellt.

Man vermischt vor dem Gebrauch 40 g der emulsionsförmigen Farbträgermasse mit 80 g der Wasserstoffperoxid-Emulsion, entsprechend einem Mischungsverhältnis von 1 : 2, trägt 120 g des gebrauchsfertigen Oxidationshaarfärbemittels, das eine Viskosität von 2100 m Pa·s aufweist, auf vollständig ergraute menschliche Haare auf und läßt es 30 Minuten lang bei Raumtemperatur einwirken. Danach wird das Haarfärbemittel mit Wasser ausgespült, das Haar mit Shampoo gewaschen, gespült und getrocknet. Das so behandelte Haar ist vom Ansatz bis zu den Haarspitzen gleichmäßig mattblond gefärbt.

### Beispiel 3: Oxidationshaarfärbemittel zur Hellerfärbung

emulsionsförmige Farbstoffträgermasse (Komponente A):

14,000 g Cetylstearylalkohol
3,000 g Glycerylstearat
2,300 g Natriumlauryldiglykolethersulfat,
28 %ige wäßrige Lösung
0,500 g Natriumsulfit, wasserfrei
0,012 g p-Phenylendiamine
0,012 g Resorcin
15,000 g Ammoniak, 25 %ige wäßrige Lösung
65.176 g Wasser
100,000 g

Wasserstoffperoxid-Emulsion (Komponente B):

4,00 g Cetylstearylalkohol
0,10 g Cholesterin
1,60 g Natriumlauryldiglykolethersulfat,
28 %ige wäßrige Lösung
24,00 g Wasserstoffperoxid, 50 %ige wäßrige Lösung
70,40 g Wasser
100,00 g

Der pH-Wert der Komponente (B) wurde mit verdünnter Phosphorsäure auf etwa 2,5 eingestellt.

Man vermischt vor dem Gebrauch 40 g der emulsionsförmigen Farbträgermasse mit 80 g der Wasserstoffperoxid-Emulsion, entsprechend einem Mischungsverhältnis von 1 : 2, trägt 120 g des gebrauchsfertigen Oxidationshaarfärbemittels, das eine Viskosität von 2300 m Pa·s aufweist, auf braune, nicht ergraute, menschliche Haare auf und läßt es 30 Minuten lang bei Raumtemperatur einwirken.

Danach wird das Haarfärbemittel mit Wasser ausgespült und das Haar getrocknet. Das so behandelte Haar ist vom Ansatz bis zu den Haarspitzen gleichmäßig blond gefärbt. Das erfindungsgemäße Mittel zum oxidativen Färben zeichnet sich durch eine besonders gute Hautverträglichkeit aus.

**Vergleichsbeispiel A: Konsistenz und Deckkraft**

Zum Vergleich der Konsistenz und der Deckkraft des erfindungsgemäßen Oxidationshaarfärbemittels mit bereits bekannten, den gleichen Gehalt an Cetylstearylalkohol aufweisenden Oxidationshaarfärbemitteln wurde ein Halbseitenversuch durchgeführt. Auf die linke Kopfhälfte von 3 Versuchsteilnehmern wurde ein übliches Oxidationshaarfärbemittel, das durch Vermischen von 20 g einer üblichen emulsionsförmigen Farbträgermasse (C) mit 40 g üblichen flüssigen Wasserstoffperoxid-Präparates (D) der nachfolgenden Zusammensetzungen hergestellt wurde, auf menschliches, ergrautes Haar aufgetragen.

übliche emulsionsförmige Farbträgermasse (C):

20,000 g Cetylstearylalkohol
0,300 g Cholesterin
6,640 g Natriumlaurylalkoholdiglykolethersulfat, 28 %ige wäßrige Lösung
0,500 g Natriumsulfit, wasserfrei
1,000 g 2,5-Diaminotoluolsulfat
0,500 g Resorcin
0,080 g m-Aminophenol
6,188 g Ammoniak, 25 %ige wäßrige Lösung
64,792 g Wasser
‾100,000‾ g

übliche Wasserstoffperoxidlösung (D):

9,000 g Wasserstoffperoxid, 50 %ige wäßrige Lösung
91.000 g Wasser
‾100,000‾ g

Der pH-Wert der Komponente (D) ist mit verdünnter Phosphorsäure auf etwa 2,5 eingestellt.

Als Vergleich dienten 60 g des erfindungsgemäßen Oxidationshaarfärbemittels gemäß Beispiel 1, die jeweils auf die rechte Kopfhälfte der Versuchsteilnehmer aufgetragen wurden.

Das erfindungsgemäße Oxidationshaarfärbemittel ist im Gegensatz zum üblichen Oxidationshaarfärbemittel, das nach dem Vermischen der beiden Komponenten (C) und (D) eine flüssige Konsistenz aufweist, bei gleichem Fettalkoholgehalt hochviskos und läßt sich mit einem Pinsel problemlos auf die Haare auftragen ohne Abzutropfen.

Beide Oxidationshaarfärbemittel wurden nach einer Einwirkzeit von 30 Minuten mit Wasser ausgespült. Das Haar wurde sodann mit Shampoo gewaschen, nachgespült und getrocknet.

Die mit dem erfindungsgemäßen Oxidationshaarfärbemittel behandelten Haare der rechten Kopfhälfte waren gleichmäßig dunkelblond gefärbt. Die vor der Färbebehandlung graue Farbe der Haare war durch das erfindungsgemäße Oxidationshaarfärbemittel deutlich besser abgedeckt worden, als durch das auf die Haare der linken Kopfhälfte aufgetragene, übliche Oxidationshaarfärbemittel.

**Vergleichsbeispiel B: Deckkraft auf grauem Haar**

Um die Deckkraft des erfindungsgemäßen Oxidationshaarfärbemittels auf grauem Haar mit der Deckkraft üblicher Oxidationshaarfärbemittel auf grauem Haar zu vergleichen, wurde ein Halbseitenversuch mit einem üblichen Oxidationshaarfärbemittel durchgeführt.

Das übliche Oxidationshaarfärbemittel wurde zunächst durch Vermischen von 20 g der emulsionsförmigen Farbträgermasse (C) mit 40 g der üblichen Wasserstoffperoxidlösung (D) hergestellt:

übliche emulsionsförmige Farbträgermasse (C):

22,000 g Cetylstearylalkohol
1,000 g Wollwachsalkohol
0,300 g Cholesterin
6,800 g Natriumlaurylalkoholdiglykolethersulfat, 28 %ige wäßrige Lösung
0,500 g Natriumsulfit, wasserfrei
1,350 g 2,5-Diaminotoluolsulfat
0,720 g Resorcin
0,056 g m-Aminophenol
0,028 g m-Phenylendiamine
7,282 g Ammoniak, 25 %ige wäßrige Lösung
59,964 g Wasser
‾100,000‾ g

übliche Wasserstofperoxidlösung (D):

9,00 g Wasserstoffperoxid, 50 %ige wäßrige Lösung
91,00 g Wasser
‾100,00‾ g Der pH-Wert der Komponente (D) wurde mit verdünnter Phosphorsäure auf etwa 2,5 eingestellt.

Jeweils 60 g dieses üblichen Oxidationshaarfärbemittels wurden sodann auf die linke Kopfhälfte von drei Versuchsteilnehmern mit grauem Haar aufgetragen.

Als Vergleich dienten 60 g des erfindungsgemäßen Oxidationshaarfärbemittels gemäß Beispiel 2, die jeweils auf die rechte Kopfhälfte aufgetragen wurden.

Beide Oxidationshaarfärbemittel wurden nach einer Einwirkungszeit von 30 Minuten mit Wasser ausgespült.

Das Haar wurde sodann mit Shampoo gewaschen, nachgespült und getrocknet. Beide Kopfhälften zeigten ein mattblond gefärbtes Haar. Der Weißanteil des ursprünglich grauen Haares war auf der rechten Seite durch das erfindungsgemäße Oxidationshaarfärbemittel jedoch deutlich besser abgedeckt.

**Vergleichsbeispiel C: Aufhellung und Hautverträglichkeit**

In einem weiteren Vergleichsversuch wurde das erfindungsgemäße Oxidationshaarfärbemittel gemäß Beispiel 3 mit einem üblichen Oxidationshaarfärbemittel hinsichtlich der Hautverträglichkeit und der aufhellenden Wirkung verglichen.

Das übliche Oxidationshaarfärbemittel wurde zunächst durch Vermischen von 20 g der üblichen emulsionsförmigen Farbträgermasse (C) mit 40 g einer üblichen Wasserstoffperoxidlösung (D) der folgenden Zusammensetzungen hergestellt:

übliche emulsionsförmige Farbträgermasse (C):

22,000 g Cetylstearylalkohol
0,200 g Cholesterin
3,000 g Glycerylstearat
5,500 g Natriumlaurylalkoholdiglykolethersulfat, 28 %ige wäßrige Lösung
0,500 g Natriumsulfit, wasserfrei
0,012 g p-Phenylendiamine
0,012 g Resorcin
15,000 g Ammoniak, 25 %ige wäßrige Lösung
53,776 g Wasser
$\overline{100,000 \text{ g}}$

übliche Wasserstoffperoxidlösung (D):

24,00 g Wasserstoffperoxid, 50 %ige wäßrige Lösung
76,00 g Wasser
$\overline{100,00 \text{ g}}$

Der pH-Wert der üblichen Wasserstoffperoxid-Lösung (D) wurde mit verdünnter Phosphorsäure auf etwa 2,5 eingestellt.

Jeweils 60 g des üblichen Oxidationshaarfärbemittels wurde auf die linke Kopfhälfte von drei Versuchsteilnehmern mit braunem, nicht ergrautem Haar aufgetragen.

Die rechte Kopfhälfte wurde mit jeweils 60 g des erfindungsgemäßen Oxidationshaarfärbemittels gemäß Beispiel 3 behandelt. Nach einer Einwirkungszeit von 30 Minuten wurde mit Wasser ausgespült und das Haar getrocknet.

Der Vergleichsversuch ergab, daß das erfindungsgemäße Oxidationshaarfärbemittel die vorher naturbraunen Haare stärker aufhellt und besser hautvertraglich ist. Das erfindungsgemäße Mittel verursachte bei keinem der Versuchsteilnehmer ein Brennen auf der Kopfhaut oder deren Rötung, während die Versuchsteilnehmer auf der linken Kopfhälfte, die mit dem üblichen Oxidationshaarfärbemittel behandelt wurden, ein Brennen der Kopfhaut feststellten.

Alle in der vorliegenden Anmeldung angegebenen Prozentangaben stellen Gewichtsprozente dar.

**Patentansprüche**

1. 2-Komponenten-Kit zur Herstellung eines Mittels zum oxidativen Färben von Haaren, bestehend aus einer emulsionsförmigen Komponente (A), welche 0,01 bis 12 Gewichtsprozent einer Entwicklersubstanz-Kupplersubstanz-Kombination und 6 bis 30 Gewichtsprozent eines Verdickergemisches enthält, mit einer emulsionsförmigen Komponente (B), welche 1 bis 18 Gewichtsprozent eines Oxidationsmittels und 3 bis 12 Gewichtsprozent eines Verdickergemischs enthält,
dadurch gekennzeichnet, daß

a) das in der Komponente (A) enthaltene Verdickergemisch, bezogen auf die Gesamtmenge dieses Verdickergemisches, 60 bis 100 Gewichtsprozent $C_{10}$- bis $C_{24}$-Fettalkohole enthält,
b) das in der Komponente (B) enthaltene Verdickergemisch, bezogen auf die Gesamtmenge dieses Verdickergemisches, 75 bis 100 Gewichtsprozent $C_{10}$- bis $C_{24}$-Fettalkohole enthält und
c) in diesem Kit die Komponente (A) zur Komponente (B) in einem Verhältnis von 1 : 1,7 bis 1 : 3 vorliegt.

2. 2-Komponenten-Kit nach Anspruch 1, dadurch gekennzeichnet, daß Komponente (A) und Komponente (B) in einem Verhältnis von 1 : 2 vorliegen.

3. 2-Komponenten-Kit nach einem der Ansprüche 1 oder 2, dadurch gekennzeichnet, daß die in den Komponenten (A) und (B) enthaltenen Fettalkohole $C_{12}$- bis $C_{20}$-Fettalkohole sind.

4. 2-Komponenten-Kit nach einem der Ansprüche 1 bis 3, dadurch gekennzeichnet, daß das in der Komponente (A) enthaltene Verdickergemisch, bezogen auf die Gesamtmenge dieses Verdickergemisches, bis zu 40 Gewichtsprozent in kosmetischen Emulsionen übliche konsistenzgebende Substanzen enthält.

5. 2-Komponenten-Kit nach einem der Ansprüche 1 bis 4, dadurch gekennzeichnet, daß das in der Komponente (A) enthaltene Verdickergemisch $C_{10}$- bis $C_{23}$-Fettsäureester mit Glycerin oder Glykolen enthält.

**6.** 2-Komponenten-Kit nach einem der Ansprüche 1 bis 5, dadurch gekennzeichnet, daß das in der Komponente (A) enthaltene Verdickergemisch, bezogen auf die Gesamtmenge dieses Verdickergemisches, 0,2 bis 25 Gewichtsprozent nicht-ionische oder anionische Emulgatoren oder Gemische derselben enthält.

**7.** 2-Komponenten-Kit nach einem der Ansprüche 1 bis 6, dadurch gekennzeichnet, daß die Komponente (A) zusätzlich 0,01 bis 6 Gewichtsprozent direktziehende Farbstoffe und mit sich selbst kuppelnde Farbstoffvorstufen enthält.

**8.** Mittel nach Anspruch 1, dadurch gekennzeichnet, daß das Oxidationsmittel Wasserstoffperoxid ist.

**9.** Mittel nach einem der Ansprüche 1 bis 8, dadurch gekennzeichnet, daß das in der Komponente (B) enthaltene Verdickergemisch, bezogen auf die Gesamtmenge dieses Verdickergemisches, 0,2 bis 25 Gewichtsprozent anionische oder nicht-ionische Emulgatoren oder deren Gemisch enthält.

## Claims

**1.** Two-component-kit for the preparation of an agent for the oxidative dyeing of hair, consisting of an emulsified component (A) containing 0.01 to 12 weight percent of a developer substance-coupler substance combination and 6 to 30 weight percent of a thickener mixture and an emulsified component (B) containing 1 to 18 weight percent of an oxidising agent and 3 to 12 weight percent of a thickener mixture, characterized in that:

   a) the thickener mixture present in component (A) contains 60 to 100 weight percent of $C_{10}$ to $C_{24}$ fatty alcohols relative to the total amount of this thickener mixture;

   b) the thickener mixture present in component (B) contains 75 to 100 weight percent of $C_{10}$ to $C_{24}$ fatty alcohols relative to the total amount of this thickener mixture; and

   c) in this two-component-kit component (A) and component (B) are present in a ratio of from 1:1.7 to 1:3.

**2.** Two-component-kit according to Claim 1, characterized in that component (A) and component (B) are present in a ratio of from 1:2.

**3.** Two-component-kit according to either of Claims 1 or 2, characterized in that the fatty alcohols present in components (A) and (B) are $C_{12}$ to $C_{20}$ fatty alcohols.

**4.** Two-component-kit according to any one of Claims 1 to 3, characterized in that the thickener mixture present in component A) contains, relative to the total amount of this thickener mixture, up to 40 weight percent of consistency-promoting substances conventional in cosmetic emulsions.

**5.** Two-component-kit according to any one of Claims 1 to 4, characterized in that the thickener mixture present in component (A) contains $C_{10}$ to $C_{23}$ fatty acid esters with glycerol or glycols.

**6.** Two-component-kit according to any one of Claims 1 to 5, characterizd in that the thickener mixture present in component (A) contains, relative to the total amount of this thickener mixture, 0.2 to 25 weight percent of non-ionic or anionic emulsifiers or mixtures thereof.

**7.** Two-component-kit according to any one of Claims 1 to 6, characterized in that component (A) additionally contains 0.01 to 6 weight percent of direct-acting dyes and self-coupling dye precursors.

**8.** Agent according to Claim 1, characterized in that the oxidising agent is hydrogen peroxide.

**9.** Agent according to any one of Claims 1 to 8, characterized in that the thickener mixture present in component (B) contains, relative to the total amount of this thickener mixture, 0.2 to 25 weight percent of anionic or non-ionic emulsifiers or a mixture thereof.

## Revendications

**1.** Dispositif („Kit") à deux composantes pour préparer une composition pour la teinture d'oxydation des cheveux être constitué par un composant (A) sous forme d'émulsion, qui contient 0,01 à 12% en poids d'une combinaison de substance révélatrice-substance de couplage et 6 à 30% en pods d'une mélange épaississant, avec un composant (B) sous forme d'émulsion, qui contient 1 à 18% en poids d'un oxydant et 3 à 12% en poids d'un mélange épaississant, caractérisé en ce que:

   a) le mélange épaississant contenu dans le composant (A), contient, par rapport à la quantité totale de ce mélange épaississant, 60 à 100% en poids d'alcools gras en $C_{10}$ à $C_{24}$,

   b) le mélange épaississant contenu dans le composant (B) contient, par rapport à la quantité totale de ce mélange épaississant, 75 à 100% en poids d'alcools gras en $C_{10}$ à $C_{24}$, et

   c) dans ce „Kit" le composant (A) par rapport au composant (B) est présent de 1:1,7 à 1:3.

2. „Kit" selon la revendication 1, caractérisé en ce que le composant (A) par rapport au composant (B) est présent de 1:2.

3. „Kit" selon la revendication 1 ou 2, caractérisé en ce que les alcools gras contenus dans les composants (A) et (B) sont des alcools gras en $C_{12}$ à $C_{20}$.

4. „Kit" selon l'une des revendications 1 à 3, caractérisé en ce que le mélange épaississant contenu dans le composant (A) contient, par rapport à la quantité totale de ce mélange épaississant, jusqu'à 40% en poids de substances donnant de la concistance usuelles dans les émulsions cosmétiques.

5. „Kit" selon l'une des revendications 1 à 4, caractérisé en ce que le mélange épaississant contenu dans le composant (A) contient des esters d'acides gras avec le glycérol ou des glycols, comportant de 10 à 23 atomes de carbone.

6. „Kit" selon l'une des revendications 1 à 5, caractérisé en ce que le mélange épaississant contenu dans le composant (A) contient, par rapport à la quantité totale de ce mélange épaississant, 0,2 à 25% en poids d'émulsionnants non-ioniques ou anioniques, ou des mélanges de ceux-ci.

7. „Kit" selon l'une des revendications 1 à 6, caractérisé en ce que le composant A) contient de plus 0,01 à 6% en poids de colorants à montée directe et des progéniteurs de colorants autocopulants.

8. Produit selon la revendication 1, caractérisé en ce que l'oxydant est l'eau oxygénée.

9. Produit selon l'une des revendications 1 à 8 caractérisé en ce que le melange épaississant contenu dans le composant (B) contient, par rapport à la quantité totale de ce mélange épaississant, 0,2 à 25% en poids d'émulsionnants anioniques on non-ioniques ou leur mélange.